Europäisches Patentamt

European Patent Office

Office européen des brevets

(11) Numéro de publication: **0 266 243**
**A1**

(12) **DEMANDE DE BREVET EUROPEEN**

(21) Numéro de dépôt: 87402173.6

(22) Date de dépôt: 30.09.87

(51) Int. Cl.⁴: **A 61 M 25/00**

(30) Priorité: 01.10.86 FR 8613681

(43) Date de publication de la demande:
04.05.88 Bulletin 88/18

(84) Etats contractants désignés: **DE FR GB IT**

(71) Demandeur: **Galtier, Claude**
**35bis, rue Lucien Berger**
**F-95130 Franconville (FR)**

**Schintgen, Jean-Marie**
**41, Avenue de Friedland**
**F-75008 Paris (FR)**

(72) Inventeur: **Galtier, Claude**
**35bis, rue Lucien Berger**
**F-95130 Franconville (FR)**

**Schintgen, Jean-Marie**
**41, Avenue de Friedland**
**F-75008 Paris (FR)**

(74) Mandataire: **Dawidowicz, Armand**
**30, Boulevard du Château**
**F-92200 Neuilly (FR)**

(54) **Implant muni d'un cathéter vasculaire ou spinal pour alimentation assistée parentérale.**

(57) L'invention concerne un implant muni d'un cathéter vasculaire ou spinal pour alimentation assistée parentérale comprenant un corps (1) en forme de disque bombé à sa partie centrale (2) et comportant un volume creux intérieur (3) relié à un cathéter (4), ledit corps (1) étant muni sur sa périphérie de perforations (15) de fixation et étant surmonté d'un tube (6) sensiblement cylindrique traversant la paroi cutanée (11). Selon l'invention, le tube (6) est fermé à son extrémité supérieure externe par un capuchon d'étanchéité clipsé interchangeable (12), ledit tube (6) étant obturé par une pastille (7') en matériau à base de silicone ou similaire et communiquant avec ledit volume creux (3) du corps (1), un joint (9) à lèvre périphérique élastique (10) étant interposé entre l'extrémité supérieure (8) dudit tube (6) et la paroi cutanée (11), ledit cathéter (4) sortant latéralement dudit corps (1).
Application: traitement par alimentation assistée parentérale.

FIG.2

EP 0 266 243 A1

## Description

Implant muni d'un cathéter vasculaire ou spinal pour alimentation assistée parentérale.

La présente invention concerne un implant muni d'un cathéter vasculaire ou spinal pour alimentation assistée parentérale.

Un tel implant est destiné à permettre l'alimentation parentérale de patients dans des traitements de chimiothéraphie, de médecine intensive, de polytraumatologie, de neurologie, etc.

On connaît par exemple un implant de ce type fabriqué par la Société VYGON aux Etats-Unis d'Amérique et vendu sous la marque "Cath-Port". Cet implant présente cependant des inconvénients de sepsis et de traumatisme cutané obligeant le praticien à modifier son action thérapeutique en fonction des problèmes suscités.

La présente invention vise à supprimer ces inconvénients des implants connus grâce à un nouvel implant de grandes fiabilité et sécurité tant pour le patient que pour le praticien, et qui apporte une plus grande souplesse d'utilisation.

A cet effet, l'implant selon l'invention est caractérisé par le fait qu'il comprend un corps en forme de disque bombé à sa partie centrale et comportant un volume creux intérieur relié à un cathéter, ledit corps étant muni sur sa périphérie de perforations de fixation et étant surmonté d'un tube sensiblement cylindrique traversant la paroi cutanée et fermé à son extrémité supérieure externe par un capuchon d'étanchéité clipsé interchangeable, ledit tube étant obturé par une pastille en matériau à base de silicone et communiquant avec ledit volume creux du corps, un joint à lèvre périphérique élastique étant interposé entre l'extrémité supérieure dudit tube et la paroi cutanée.

L'implant selon l'invention permet une implantation simple et non traumatisante et assure une fixation durable grâce à la forme de disque du corps et des orifices de fixation. Avantageusement, les orifices de fixation sont oblongs, ce qui permet une orientation de l'implant par rapport au vaisseau à cathétériser, avant fixation par couture, pour éviter des contraintes inacceptables sur le cathéter.

De préférence, le capuchon d'étanchéité comporte, sur une portion d'environ 180° d'angle de sa périphérie, une empreinte d'enclipsage coopérant avec une empreinte complémentaire de la partie haute du tube et, à l'opposé de ladite empreinte d'enclipsage, un crochet coopérant avec un crochet complémentaire de la partie haute du tube pour former une articulation démontable. Avantageusement, le capuchon comporte un marquage, par exemple une couleur, de repérage du type de vaisseau auquel est relié le cathéter et/ou d'indication du traitement subi par le patient.

Dans une forme de réalisation particulièrement préférée, l'implant est radiotransparent. De préférence, la partie creuse du tube est tronconique. Avantageusement, le cathéter est muni, sur sa partie distale, d'ergots de maintien espacés. De préférence, ledit cathéter est muni dans sa paroi d'un filet radio-opaque.

L'invention sera bien comprise à la lecture de la description suivante faite en se référant au dessin annexé dans lequel:

    - la figure 1 est une vue schématique en perspective d'un implant selon l'invention, sans joint et sans capuchon, et

    - la figure 2 est une vue schématique en coupe axiale de l'implant de la figure 1 après implantation.

L'implant selon l'invention est formé d'un corps 1 en forme de disque avec une portion centrale bombée 2 comprenant une chambre 3 d'évacuation de substances injectables reliée à un cathéter 4 muni, sur sa partie distale, d'ergots de maintien espacés 5. De préférence, le cathéter 4 comporte, dans sa paroi, un filet radio-opaque permettant une visualisation immédiate de la mise en place per-opératoire, sans avoir à utiliser une substance de contraste. Ceci a pour avantages

    1°- d'éviter les risques inhérents à l'utilisation de ce type de produit (choc anaphylactique plus ou moins grave);

    2°- d'éviter le lavage systématique après l'injection de ce type de produit qui cristalise très rapidement et qui aurait pour effet d'obstruer l'embouchure proximale du cathéter, voire la chambre d'évacuation;

    3°- de réduire la durée de mise en place de l'ensemble, sous contrôle (scopie, ampli de brillance), ce qui est un bénéfice non négligeable lorsque l'on sait que les éventuels porteurs de ce type d'appareil ne sont pas en très bonne santé, et surtout si l'implantation se fait sous anesthésie locale ou loco-régionale.

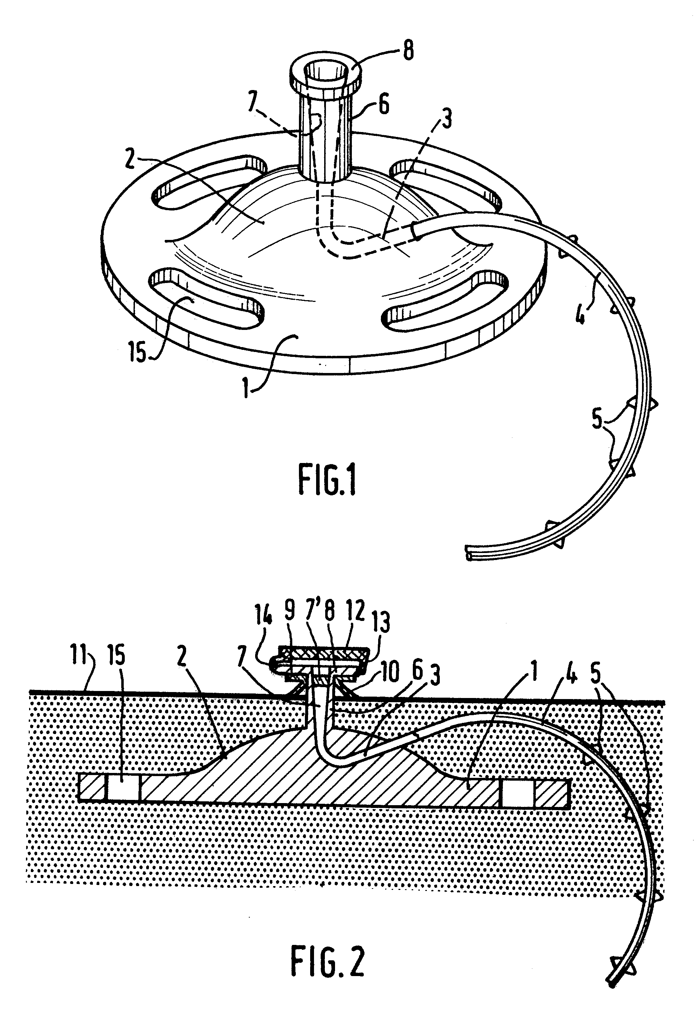

La partie bombée 2 du corps 1 est surmontée par un tube 6 à intérieur 7 tronconique et terminé par une collerette 8. La conicité de la partie interne 7 du tube 6 a pour but de minimiser la quantité résiduelle du produit injecté, de faciliter son écoulement vers le cathéter, surtout si la substance injectée ne suppose pas de lavage terminal. Un joint 9 (figure 2) muni d'une lèvre périphérique élastique 10 est logé contre l'épaulement inférieur de la collerette 8 de manière que la lèvre 10 s'appuie de manière étanche sur le plan cutané 11. Le tube 6 est obturé par une pastille 7' en matériau à base de silicone ou similaire.

Un capuchon 12 comporte sur 180° de sa périphérie une lèvre d'encliquetage 13 coopérant avec une lèvre complémentaire de la collerette 8. A l'opposé, le capuchon est muni d'un crochet 14 coopérant avec un crochet complémentaire de la collerette 8 pour former une articulation. Le capuchon 12 peut ainsi facilement être remplacé en cas de perte ou de détérioration et le joint 9, par sa lèvre 10, assure une pression suffisante lors de l'introduction et de l'extraction de l'aiguille tout en garantissant une totale étanchéité au niveau du plan sous-cutané.

La périphérie du corps 1 est munie de perforations oblongues 15, courant sur environ 45° d'angle, autorisant l'orientation angulaire de l'implant avant fixation.

L'implant est réalisé par exemple en polyuréthane pour obtenir les qualités mécaniques et d'atraumatisme voulues.

Le cathéter, avantageusement, est interchangeable pour permettre au praticien de choisir le type et les dimensions du cathéter convenant au type d'intervention qu'il entreprend.

La partie supérieure du tube 2 peut être taraudée pour recevoir une seringue sans aiguille du type appelé "cône luer lock". La position basse de la pstille 7' ne gêne pas une telle utilisation.

## Revendications

1.- Implant muni d'un cathéter vasculaire ou spinal pour alimentation assistée parentérale comprenant un corps (1) en forme de disque bombé à sa partie centrale (2) et comportant un volume creux intérieur (3) relié à un cathéter (4), ledit corps (1) étant muni sur sa périphérie de perforations (15) de fixation et étant surmonté d'un tube (6) sensiblement cylindrique traversant la paroi cutanée (11), caractérisé par le fait que ledit tube (6) est fermé à son extrémité supérieure externe par un capuchon d'étanchéité clipsé interchangeable (12), ledit tube (6) étant obturé par une pastille (7') en matériau à base de silicone ou similaire et communiquant avec ledit volume creux (3) du corps (1), un joint (9) à lèvre périphérique élastique (10) étant interposé entre l'extrémité supérieure (8) dudit tube (6) et la paroi cutanée (11), ledit cathéter (4) sortant latéralement dudit corps (1).

2.- Implant selon la revendication 1, caractérisé par le fait que les orifices de fixation (15) sont oblongs.

3.- Implant selon l'une des revendications 1 et 2, caractérisé par le fait que le capuchon d'étanchéité (12) comporte, sur une portion d'environ 180° d'angle de sa périphérie, une empreinte d'enclipsage (13) coopérant avec une empreinte complémentaire de la partie haute (8) du tube (6) et, à l'opposé de ladite empreinte d'enclipsage (13), un crochet (14) coopérant avec un crochet complémentaire de la partie haute (8) du tube (6) pour former une articulation démontable.

4.- Implant selon l'une des revendications 1 à 3, caractérisé par le fait que le capuchon (12) comporte un marquage, par exemple une couleur, de repérage du type de vaisseau auquel est relié le cathéter (4) et/ou d'indication du traitement subi par le patient.

5.- Implant selon l'une des revendications 1 à 4, caractérisé par le fait qu'il est radiotransparent.

6.- Implant selon l'une des revendications 1 à 5, caractérisé par le fait que ledit cathéter (4) est muni dans sa paroi d'un filet radio-opaque.

7.- Implant selon l'une des revendications 1 à 6, caractérisé par le fait que le cathéter (4) est muni, sur sa partie distale, d'ergots de maintien (5) espacés.

8.- Implant selon l'une des revendications 1 à 7, caractérisé par le fait que la partie creuse (7) du tube (6) est tronconique.

9.- Implant selon l'une des revendications 1 à 8, caractérisé par le fait que le cathéter (4) est interchangeable.

10.- Implant selon l'une des revendications 1 à 9, caractérisé par le fait que l'extrémité supérieure du tube (6) comporte un taraudage pour le montage d'une seringue sans aiguille.

0266243

FIG.1

FIG.2

**Office européen des brevets**

**RAPPORT DE RECHERCHE EUROPEENNE**

Numero de la demande

EP 87 40 2173

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | GB-A-2 056 282 (BENTLEY)<br>* Description; figures 5,14 *<br>--- | 1,4,8,9 | A 61 M 25/00 |
| X | EP-A-0 174 802 (W.L. GORE)<br>* Résumé; figures *<br>--- | 1,8,9 | |
| A | FR-A-2 294 718 (SHERWOOD)<br>* Page 4, lignes 33-36; figure 4 *<br>--- | 6 | |
| A | US-A-4 306 545 (M. IVAN)<br>* Figure 3 *<br>--- | 2 | |
| A | US-A-4 425 119 (BERGLUND)<br>* Colonne 7, lignes 61-68; figure 2 *<br>----- | 10 | |

**DOMAINES TECHNIQUES RECHERCHES (Int. Cl.4)**

A 61 M

Le présent rapport a été établi pour toutes les revendications

| Lieu de la recherche | Date d'achèvement de la recherche | Examinateur |
|---|---|---|
| LA HAYE | 07-01-1988 | VANRUNXT J.M.A. |